# EUROPEAN PATENT SPECIFICATION

(11) **EP 1 766 396 B1**
(45) Date of publication and mention of the grant of the patent: **11.08.2010**
(21) Application number: 05756588.9
(22) Date of filing: 06.06.2005
(51) Int. Cl.: A61K 39/395, G01N 33/53, A61K 39/00, A61K 45/00, C07K 16/18

(54) **METHOD OF PASSIVE IMMUNIZATION AGAINST DISEASE OR DISORDER CHARACTERIZED BY AMYLOID AGGREGATION WITH DIMINISHED RISK OF NEUROINFLAMMATION**
VERFAHREN ZUR PASSIVEN IMMUNISIERUNG GEGEN EINE DURCH AMYLOIDAGGREGATION GEKENNZEICHNETE KRANKHEIT ODER ERKRANKUNG MIT VERMINDERTEM NERVENENTZÜNDUNGSRISIKO
PROCEDE D'IMMUNISATION PASSIVE CONTRE DES MALADIES OU DES TROUBLES CARACTERISE(E)S PAR AGREGATION AMYLOIDE A RISQUE DIMINUE DE NEUROINFLAMMATION

(30) Priority: 07.06.2004 US 577202 P
(43) Date of publication of application: 28.03.2007
(73) Proprietor: Ramot at Tel-Aviv University Ltd., 61392 Tel Aviv (IL)
(72) Inventor: SOLOMON, Beka, 46399 Herzlia Pituach (IL); REBE, Sabina, 77622 Ashdod (IL)
(74) Representative: Andersson, Inga-Lill
(86) International application number: PCT/US2005/019617
(87) International publication number: WO 2005/120571

(56) References cited:
- WO-A-03/016466
- WO-A-03/086310
- WO-A-03/101485
- US-A1- 2004 192 898
- US-A1- 2004 241 164
- US-A1- 2004 248 197
- US-A1- 2005 054 832
- US-A1- 2005 112 700
- US-B1- 6 284 536
- S. RADAEV & P. SUN: "Recognition of IgG by Fcgamma receptor." THE JOURNAL OF BIOLOGICAL CHEMISTRY, vol. 276, no. 19, 11 May 2001 (2001-05-11), pages 16478-16483, XP002214196 U.S.A.
- L-F. LUE & D. WALKER: "Modeling Alzheimer's disease immune therapy mechanisms: Interactions of human postmortem microglia with antibody-opsonized amyloid beta peptide." JOURNAL OF NEUROSCIENCE RESEARCH, vol. 70, no. 4, 15 November 2002 (2002-11-15), pages 599-610, XP008078719
- B. BACSKAI ET AL.: "Non-Fc-mediated mechanisms are involved in clearance of amyloid-beta in vivo by immunotherapy." THE JOURNAL OF NEUROSCIENCE, vol. 22, no. 18, 15 September 2002 (2002-09-15), pages 7873-7878, XP002986009
- S. REBE & B. SOLOMON: "Deglycosylation of anti-beta amyloid antibodies inhibits microglia activation in BV-2 cellular model." AMERICAN JOURNAL OF ALZHEIMER'S DISEASE AND OTHER DEMENTIAS, vol. 20, no. 5, September 2005 (2005-09), pages 303-313, XP008078061 U.S.A.
- WRIGHT A.; MORRISON S.: 'Effect of CH2-associated carbohydrate structure on the functional properties and in vivo fate of chimeric mouse-human immunoglobulin.' THE JOURNAL OF EXPERIMENTAL MEDICINE vol. 180, September 1994, pages 1087 - 1096
- T. SHANTA RAJU "Impact of Fc glycans on antibody functions and stability" In THE NEW YORK ACADEMY OF SCIENCES, 22 May 2007 (2007-05-22), page 3.

## Description

### BACKGROUND OF THE INVENTION

### Field of the Invention

The present invention relates to passive immunization against a disease disorder characterized by amyloid aggregation using unglycosylated antibodies capable of avoiding neuroinflammation.

### Description of the Related Art

Methods for the prevention or treatment of diseases characterized by amyloid aggregation in a patient have been proposed which involve causing antibodies against a peptide component of an amyloid deposit to come into contact with aggregated or soluble amyloid. See WO99/27944 of Schenk and U.S. patent 5,688,651 of Solomon. The antibodies may be caused to come into contact with the soluble or aggregated amyloid by either active or passive vaccination. In active vaccination, a peptide, which may be an entire amyloid peptide or a portion thereof, is administered in order to raise antibodies in vivo, which antibodies will bind to the soluble and/or the aggregated amyloid. Passive vaccination involves administering antibodies specific to the amyloid peptide directly. These procedures are preferably used for the treatment of Alzheimer's disease by diminishing the amyloid plaque or slowing the rate of deposition of such plaque.

It has been reported that clinical trials had been undertaken by Elan Corporation and Wyeth-Ayerst Laboratories of a vaccine to test such a process. The compound being tested was AN-1792. This product has been reported to be a form of β-amyloid 42. However, in February of 2002, the two companies announced that the vaccine study had been halted after more than a dozen participants developed severe brain inflammation. In view of the promising prospects of such an immunotherapy program, particularly in light of the animal data set forth in WO99/27944 and Schenk et al (1999), it would be of great benefit to find a way to allow the clinical trials of this immunotherapeutic method to continue without the risk of brain inflammation.

Antibody-antigen complexes initiate the inflammatory response and are central to the pathogenesis of tissue injury. The accepted model of inflammation is one in which antibodies bind their antigen, forming immune complex, which in turn binds and activates the complement by means of the "classical pathway" (Clynes et al, 1995).

The classical model for this immunopathological cascade, the Arthus reaction, was reinvestigated with a murine strain deficient in Fc receptor expression (Sylvestre et al, 1994). Despite normal inflammatory responses to other stimuli, the inflammatory response to immune complexes was markedly attenuated. These results suggest that the immune complex-triggered inflammation is initiated by cell-bound Fc receptors and is then amplified by cellular mediators and activated complement. These results further redefine the inflammatory cascade and may offer other approaches for the study and treatment of immunological injury.

Cell membrane receptors specific for the Fc portion of immunoglobulin (FcR) play an important role in immunity and resistance to infection, providing a system that couples antibody-antigen interaction with cellular effector mechanisms. Distinct cell membrane FcRs have been described for all classes of immunoglobulins. The FcRs comprise a multi-membered family of structurally homologous but distinct receptors and are expressed on the vast majority of leukocytes. The diversity of these receptors is reflected in a wide variety of biological responses immediately upon their binding of IgG-antigen complexes, including phagocytosis, endocytosis, antibody-dependent cell-mediated cytotoxicity (ADCC), release of inflammatory mediators and regeneration of B-cell function (Clynes et al, 1995).

In addition to a long list of inflammatory pathways thought to be involved in AD, there are particular inflammatory pathways that are activated specifically following Fc receptor stimulation that may, paradoxically, exacerbate brain inflammation and AD-related neurodegeneration in the process of scavenging Aβ.

Immunization of animals or humans results in the production of anti-Aβ antibodies that trigger brain resident microglial cells to clear Aβ from the brain via cell surface Fc receptors which may increase toxic free radical production in microglial cells.

Microglia are considered the resident immune cells of the central nervous system (CNS). In the mature brain and under physiological conditions, resting microglia adopt the characteristic ramified morphological appearance and serve the role of immune surveillance and host defense. Microglia, however, are particularly sensitive to changes in their microenvironment and readily become activated in response to infection or injury.

Microglial activation is frequently observed in the pathogenesis of neurodegenerative diseases, such as Alzheimer's disease, Parkinson's disease, multiple sclerosis, AIDS dementia complex and amyotrophic lateral sclerosis. In addition, glia, especially microglia, become activated (a process termed reactive gliosis) following an initial wave of neuronal death resulting from traumatic injury, exposure to neurotoxins, and ischemia in the brain. Activated microglia up-regulate a variety of surface receptors, including the major histocompatibility complex and complement receptors. Upon activation, microglia secrete a range of immune regulatory peptides as cytokines and non-specific inflammatory mediators and become phagocytic, thus representing the latent scavenger cels of the CNS (Liu et al., 2001). The majority of factors produced by activated microglia, however, are pro-inflammatory and neurotoxic. These include the cytokines such as tumor necrosis factor-α (TNFα) and interleukin-1β (IL-1β), free radicals such as nitric oxide (NO) and superoxide, fatty acidmetabolites such as eicosanoids, and quinolinic acid. Studies using cell culture and animal models have demonstrated that excessive quantities of individual factors produced by activated microglia can be deleterious to neurons (Boje and Arora, 1992; Chao et al., 1992; McGuire et al., 2001). The involvement of microglial activation in the pathogenesis of several neurodegenerative diseases was initially postulated based on the postmortem analysis of the brains of patients with Alzheimer's disease (AD) and Parkinson's disease (PD). Microglial activation may play a pivotal role in the initiation and progression of several neurodegenerative diseases. Inhibition of microglial activation, therefore, would be an effective therapeutic approach to alleviating the progression of diseases such as AD and PD.

The induction of an antibody response to amyloid beta (Aβ) peptide has become a strategy for the treatment of Alzheimer's disease (AD), having proved effective in reducing the plaque burden in transgenic mice that develop Aβ plaques similar to human AD patients (Wilcock et al., 2004). The mechanisms by which immunotherapy acts remain unclear. Suggested mechanisms include microglial-mediated phagocytosis (Schenk et al. 1999; Wilcock et al. 2003; Wilcock et al. 2001; and Wilcock et al. 2004), disaggregation of amyloid deposits (Solomon et al. 1997 ; Wilcock et al. 2003; and Wilcock et al. 2004), and removal of Aβ from the brain by binding circulating Aβ in plasma with the anti-Aβ antibodies, resulting in a concentration gradient from brain to plasma (DeMattos et al. 2001; Dodart et al. 2002; and Lemere et al. 2003). Clearance of Aβ, caused partly by the interaction of immunoglobulin Fc receptor-expressing microglia and specific antibody-opsonized Aβ deposits (Bard et al. 2000), stimulates Fc receptor-mediated phagocytosis but also results in inflammatory activation of these cells (Lue et al., 2002). Activated microglia up-regulate a variety of surface receptors, including the major histocompatibility complex, F4/80 cell surface antigen and/or complement receptors (Castano et al. 1996; and Chao et al. 1994). Most of the factors produced by activated microglia are, however, pro-inflammatory and neurotoxic (Boje et al., 1992; and Chao et al. 1992). Consequently, interaction of microglia with antibody-antigen complexes could exacerbate existing inflammation in the brains of AD patients.

Indeed, the immunotherapeutic approach applied in the first clinical trials in AD patients with AN1792 (fibrillar Aβ 1-42) led to inflammation, reinforcing the importance of clarifying the nature and mechanisms of the observed adverse events in the CNS.

Two recent reports involving a subset of patients that were enrolled in the trial have shed some light on potential therapeutic values, as well as possible drawbacks induced by this strategy.

In the postmortem case, there was clear evidence of decreased amounts of Aβ plaques in neocortex regions compared with non-immunized AD patients. In some regions that were devoid of Aβ plaques, AβP immunoreactivity was associated with T-cell infiltrates in the CNS, activated microglia, and low titer of AβP antibodies (Nicoll et al., 2003). Activation of microglial inflammatory mediators that may participate in Fc receptors-mediated opsonization or scavenging of Aβ plaques have detrimental effects or even lead to acceleration of neurodegeneration (Das et al., 2002).

Indeed, *in vitro* studies with rodent microglia incubated with antibody-opsonized Aβ demonstrated enhanced Fcγ receptor-mediated phagocytosis, compared with microglia incubated with Aβ alone (Lue et al., 2002).

The Fc region of the antibodies is the interaction site of a number of effector molecules, (Radaev et al., 2001). The N-linked oligosaccharides on the heavy chains of immunoglobulins are known to play a role in effector functions, including complement activation and FcR binding on effector cells (Nose et al., 1983; and Jefferis et al. 1995).

Recently determined crystal structures of the complex between immunoglobulin constant regions (Fc) and their Fc-respective receptors (FcR) revealed detailed molecular interactions. Of particular interest is the contribution of glycosylation at Asn297 of the C_{H}2 domain of IgG to receptor recognition.

Carbohydrate moieties are found outside the receptor Fc interface in all receptor Fc complex structures. The removal of carbohydrates resulted in reduced receptor binding to the Fc (Collin et al. 2002).

The IgG molecule contains carbohydrate at conserved position Asn 297 in the Fc region. It is a single N-linked bi-antennary structure which is buried between the C_{H}2 domains, forming extensive contacts with amino acid residues within the domain (Radaev, 2001). Multiple non-covalent interactions between the oligosaccharide and the protein result in reciprocal influences of each on the conformation of the other. Effector mechanisms mediated through various Fc receptors (FcγRI, FcγRII, FcγRIII) of the complement pathway and Ciq are severely compromised or aborted for aglycosylated or deglycosylated forms of IgG (Radaev, 2001). This interaction can stimulate Fc receptor-mediated phagocytosis, but also results in inflammatory activation of these cells. Consequently, interaction of microglia with antibody-antigen complexes could exacerbate the existing inflammation in the brains of AD patients.

Completely deglycosylated IgGbinds significantly less efficiently to FcR than IgG. Effector mechanisms, mediated through various Fc receptors (FcγRI, FcγRII, FcγRIII) of the complement pathway and C1q, are compromised or aborted for aglycosylated or deglycosylated forms of IgG (Radaev et al., 2001). Removal of these oligosaccharides most likely changes the structure of the Fc portion from an open conformation to a more closed conformation and deglycosylated IgG had a 10- to 15-fold lower affinity for FcγRIII than intact IgG (Radaev et al., 2001). However, N-linked oligosaccharides did not appear to be important for the ability of IgG to activate the alternative complement pathway.

WO 03/086310 of Solomon, which is a publication of one of the present inventors, discloses that the problem of increased risk of brain inflammation as a result of induced autoimmune response is addressed by eliminating the inflammation pathway initiated by binding of an immune complex to an Fc receptor. It was realized that the brain inflammation that caused the cessation of the clinical trials for AN-1792 was most likely caused by the inflammatory reaction initiated by binding of the immune complex to Fc receptors. This immune reaction could be stopped before it begins by one of two techniques. The first such technique is to block the Fc receptors prior to commencing the immunotherapy, such as by administering a large dose of IVIg, i.e., human intact intravenously administered immunoglobulin.

Intravenous immunoglobulins (IVIg) have become an established component of immunomodulatory therapy in neurological autoimmune diseases, including inflammatory diseases of the central nervous system (CNS) (van der Meché and van Doorn, 1997; Dalakis, 1999; Stangel et al, 1999). WO 03/086310 discloses that IVIg can be used as a preventive step prior to immunotherapy designed to cause antibodies against amyloid-β to come into contact with aggregated or soluble amyloid-β in vivo, regardless of whether the antibodies are directly administered or generated *in vivo* by administering an antigenic peptide, such as an amyloid peptide.

The second method to avoid binding of the immune complex to Fc receptors is to use antibodies that are devoid of Fc regions. Thus, rather than generating intact antibodies in vivo by active immunization, one would administer antibodies by passive immunization but using antibodies devoid of Fc regions. Examples of antibodies devoid of Fc regions include Fab, F(ab)₂ and/or scFv antibodies. Such antibodies will still bind to the amyloid or amyloid plaque, but the immune complexes will not start the inflammation sequence because they will not bind to Fc receptors.
B. BACSKAI et. al. disclose in "Non-Fc-mediated mechanisms are involved in clearance of amyloid beta in vivo by immunotherapy" THE JOURNAL OF NEUROSCIENCE, vol. 22, no. 18, 15 September 2002, pp. 7873-7878, XP002986009, antibody-mediated clearance of amyloid-beta deposits in vivo by immunotherapy using non-Fc-mediated disruption of plaques in a mouse model.
In WO 03/016466 A (ELI LILLY AND COMPANY) 27 February 2003, it is disclosed that variants of anti-beta amyloid mAb 266 lacking an N-glycosylation site within the VH-CDR2 have a greater affinity for beta amyloid than the normal, glycosylated mAb.

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### SUMMARY OF THE INVENTION

The present invention provides the use of an unglycosylated antibody against beta amyloid peptide for manufacturing a medicine for preventing, inhibiting or treating a disease or disorder characterized by amyloid aggregation, where the antibody in unglycosylated, i.e., deglycosylated or aglycosylated, in the Fc region.
The present invention also relates to an unglycosylated antibody against beta amyloid peptide for use in preventing, inhibiting or treating a disease or disorder, which is characterized by amyloid aggregation, wherein said antibody is unglycosylated in the Fc region, and diminishes the risk of neuroinflammation. The present invention reduces the risk of neuroinflammation that may be associated with passive administration of intact native antibodies.

### BRIEF DESCRIPTION OF THE DRAWINGS

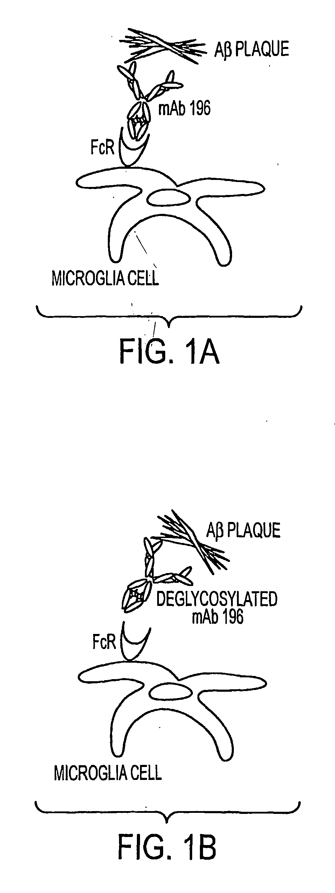

Figures 1A and 1B are schematic representations of the effect of antibody deglycosylation and its role in immmotherapeutic strategy against Alzheimer's disease. Fig. 1A represents unmodified antibody - FcR mediated pro-inflammatory mechanism, while Fig. 1B represents the proposed inhibitory effect on the pro-inflammatory mechanism. Fc region of the anti Aβ antibody is deglycosylated in order to reduce its binding to FcR on microglial cell towards beneficial effects of immunotherapy against Aβ. The drawing is not to scale.

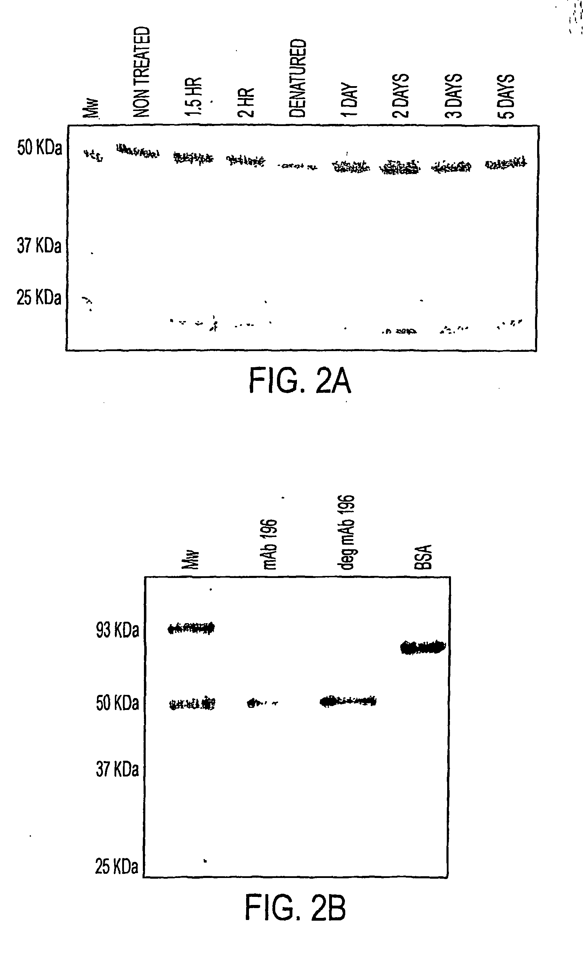

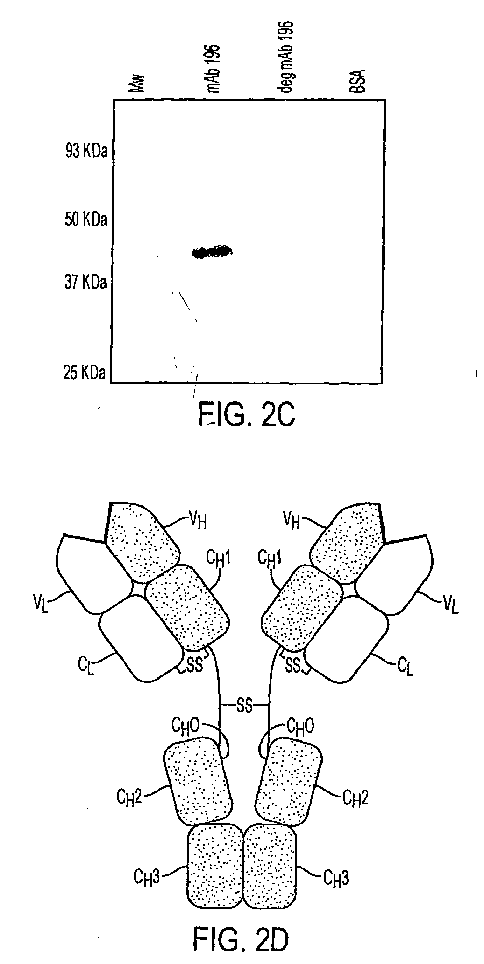

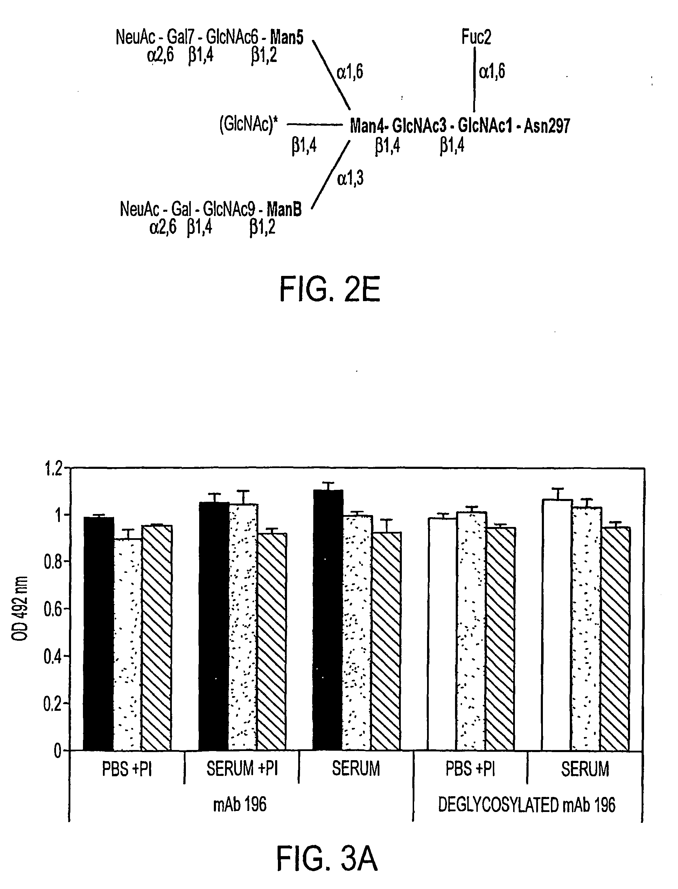

Figures 2A-2E show the deglycosylation of native mAb 196. In Fig. 2A, mAb 196 was deglycosylated via enzymatic cleavage with PNGase. Deglycosylation reaction was sampled at different time points and compared to mAb 196 that was fully deglycosylated under denaturing conditions, and deglycosylation was analyzed by 10% SDS PAGE (Figs. 2A and 2B). Glycosylated mAb 196, deglycosylated mAb196 and Bovine Serum Albumin (BSA), as negative control, were blotted onto PVDF membrane and incubated with a lectin, Concanavalin A (Con A), that binds to core mannose of the glycan (Fig. 2C). 3 µg per lane of each sample was applied to 10% bis-acrylamide gel and stained with Coomassie blue. MAb 196, under denaturating conditions, showed two separate bands: heavy and light chains at 50kDa and 25kDa, respectively. After 1.5 hr treatment with PNGase, the 50kDa band split in two, the lower one representing deglycosylated heavy chain. MAb 196, deglycosylated under denaturing conditions, displayed only one band at 50kDa. Longer incubations with PNGase yielded an increase in deglycosylation product. After 5 days incubation with PNGase, the mAb displayed one lower band at 50kDa, indicating full deglycosylation. For the Con A blot, 3 µg per lane of each sample was applied to two 12% bis-acrylamide gels. One gel was stained with coomassie blue (Fig. 2B), and the other was blotted onto PVDF membrane and reacted with Con A (Fig. 2C). The Coomassie blue stained gel showed BSA and heavy and light chains of native mAb 196 and deglycosylated mAb 196. Membrane reacted with Con A showed only one band at 50kDa corresponding to the heavy chain of native mAb 196. Con A did not bind to BSA or to deglycosylated mAb 196. Fig. 2D is a schematic representation of an IgG molecule. Fc glycan is indicated as CHO moiety between two C_{H}2 domains. The carbohydrate sequence attached at Asn297 of human IgG1-Fc is presented in Fig. 2E.

Figures 3A-3D show *in vitro* stability and antigen recognition functions of deglycosylated mAb 196. In Fig. 3A, native (black) and deglycosylated (white) mAb 196 were incubated in serum/serum + Protease Inhibitors (PI)/PBS + Protease Inhibitors (PI) for different periods of time: 0 hours (block), 2 hours (horizontal stripes) and 7 days (diagonal stripes). After incubation, samples were applied to 96 well ELISA plate coated with AβP 1-16. Bound antibody was detected by horseradish peroxidase conjugated goat anti-mouse IgG antibody. Measured OD 492nm corresponds to the amount of antibody bound to the antigen. Error bars represent Standard Deviation in OD 492nm values calculated from three independent experiments. No significant differences in OD492 were observed between native and deglycosylated mAb196.

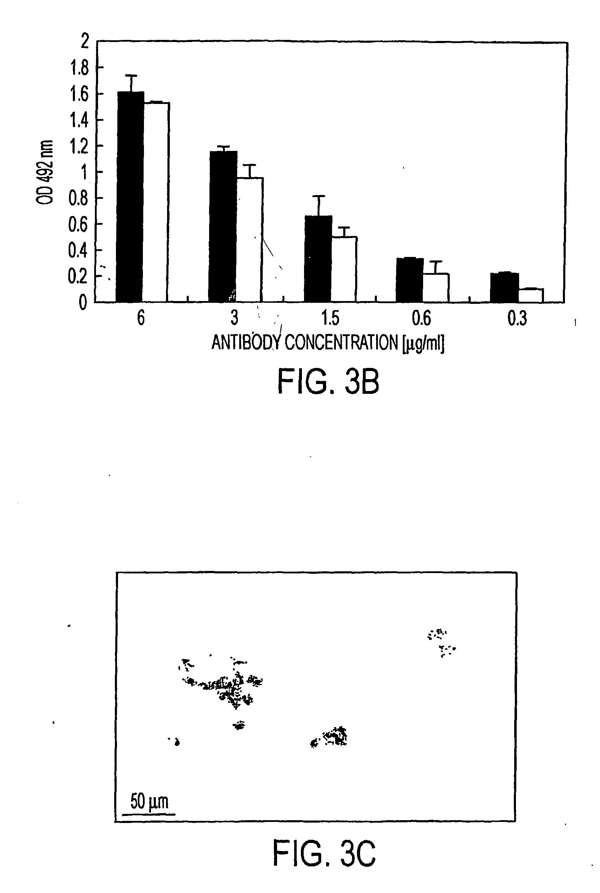

In Fig. 3B native (black) and deglycosylated (white) mAb 196 were applied at different concentrations to 96 well ELISA plate coated with AβP 1-16. Bound antibody was detected as described above. No significant changes in OD492 nm values were observed between native and deglycosylated form of mAb 196, except for the lowest antibody concentration (0.3 mg/ml).

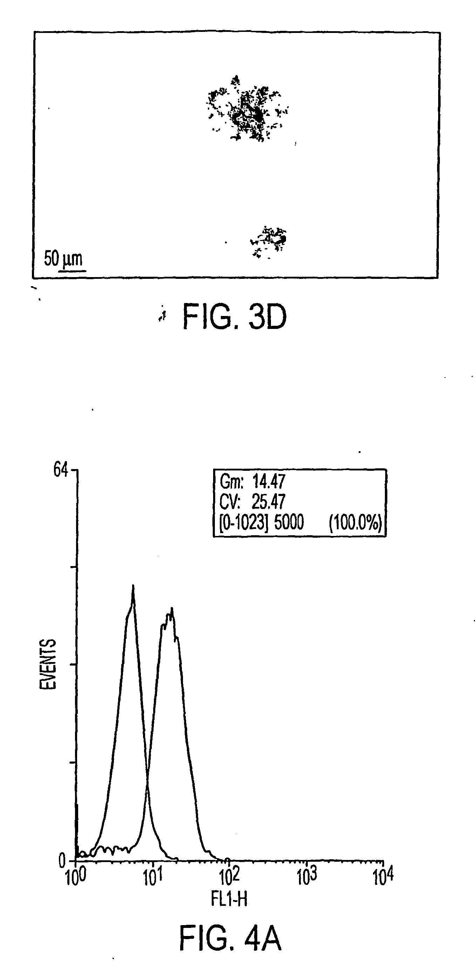

Native (Fig. 3C) and deglycosylated (Fig. 3D) mAb 196 were applied at the same concentration of 1mg/ml to coronal brain sections of APP transgenic mice (Tg2576). Staining of AβP plaques induced by bound antibody was developed with DAB chromogen (brown color). Equally stained AβP plaques were observed for both native and deglycosylated forms of mAb 196.

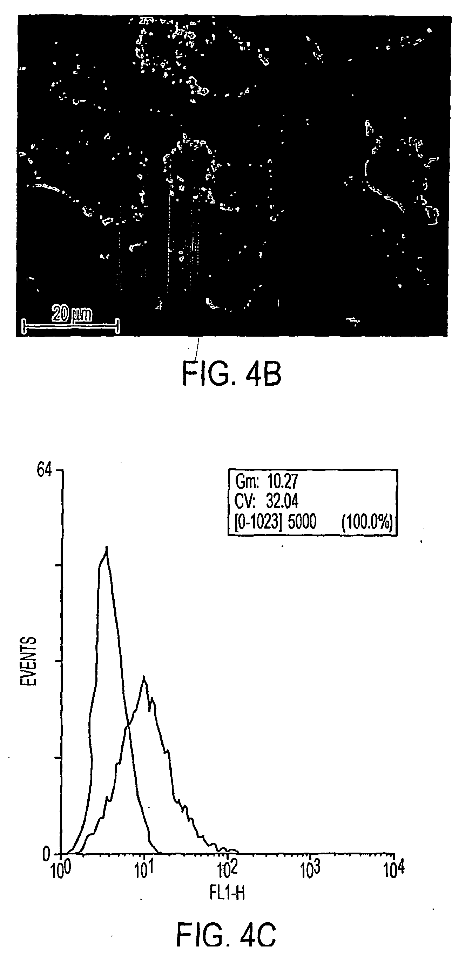

Figures 4A-4F show binding of deglycosylated mAb 196 to Fcγ receptors on murine microglial cells. In Fig. 4A, BV-2 cells were labeled with rat α mouse FcγRII/III (CD16/32) and biotin conjugated goat α rat IgG/ avidin FITC and subjected to FACS analysis (right curves). As negative control cells were incubated with secondary antibody alone (left curves). BV-2 cells labeled with rat α mouse FcgRII/III (CD16/32) displayed a peak shift on the fluorescence scale, Gm 14.47, indicating an increase in mean cell fluorescence in comparison to the base line, Gm 4.40, cells incubated with secondary antibody alone. Scanning laser confocal microscope image of BV-2 cells labeled with rat α mouse FcgRII/III (CD16/32) shows positive, membrane associated staining, degree of cell surface labeling varies from cell to cell (Fig. 4B). Bar in Fig. 4B corresponds to 20 mm.

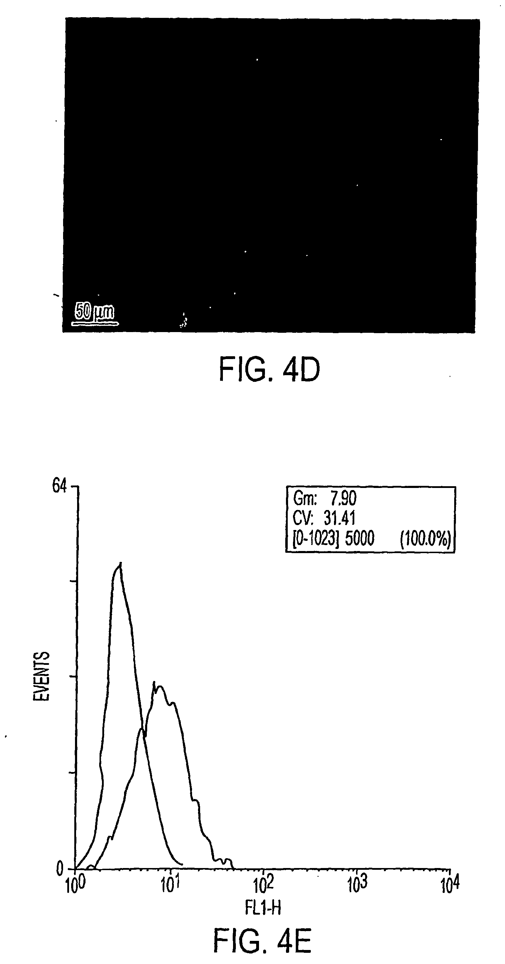

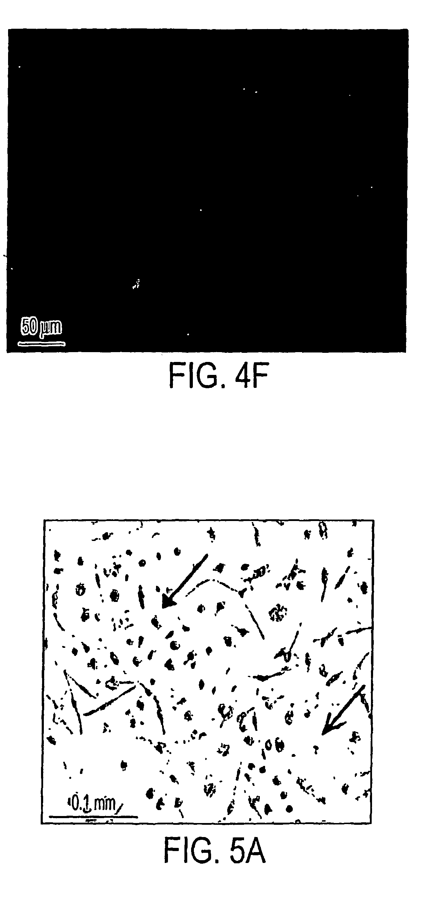

Immunocomplex of deglycosylated mAb 196 and Aβ 1-42 was added to live BV-2 cultured cells. Bound immunocomplex was detected by Cg3 conjugated goat α mouse IgG. Cells were visualized using fluorescent microscope (Figs. 4D and 4F) and FACS (Figs. 4C and 4E). BV-2 cells incubated with immunocomplex of deglycosylated mAb 196 and pre-aggregated AαP 1-42 at final antibody concentration of 10mg/ml (Fig. 4E) displayed smaller peak shift on the fluorescence scale, Gm 7.9, indicating an increase in mean cell fluorescence in comparison to a corresponding native immunocomplex, Gm 10.27 (Fig. 4C). Cells incubated with secondary antibody alone (Figs. 4C and 4E, left curves) exhibited a very low mean cell fluorescence of 3.26. Similarly, positive staining was observed under a fluorescent microscope of BV-2 cells incubated with native immunocomplex (Fig. 4D) and a decline in cell-associated fluorescence when deglycosylated immunocomplex (Fig. 4F) was introduced.

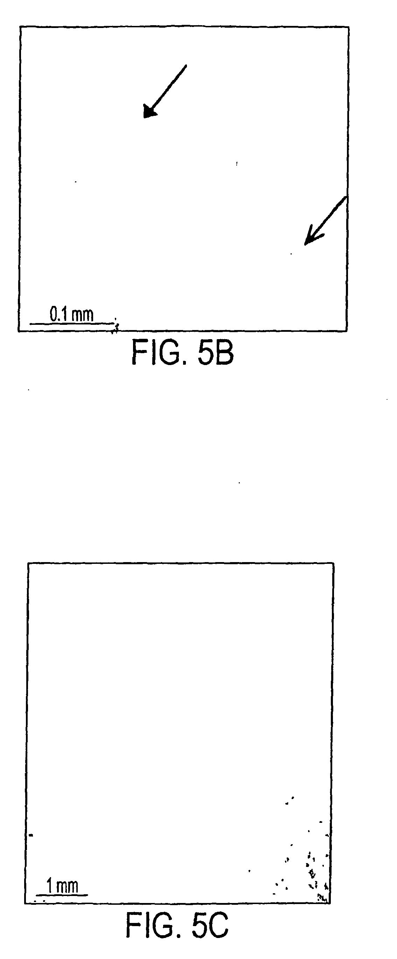

Figures 5A-5H show F4/80 immunohistochemistry of microglial migration response to antibody opsonized Aα spot. BV-2 cells grown in serum free medium were fixed and immunostained with rat anti-mouse F4/80 antibody. Color was developed using DAB chromogen (golden brown). Stained cells were visualized under a light microscope both with phase contrast (Fig. 5A) and without phase contrast (Fig. 5B). All cells displayed positive staining with anti-F4/80 antibody. Phagocytic microglial cells (Figs. 5A and 5B, solid arrow) were more intensely stained than ramified microglial cells (Figs. 5A and 5B, hollow arrow).

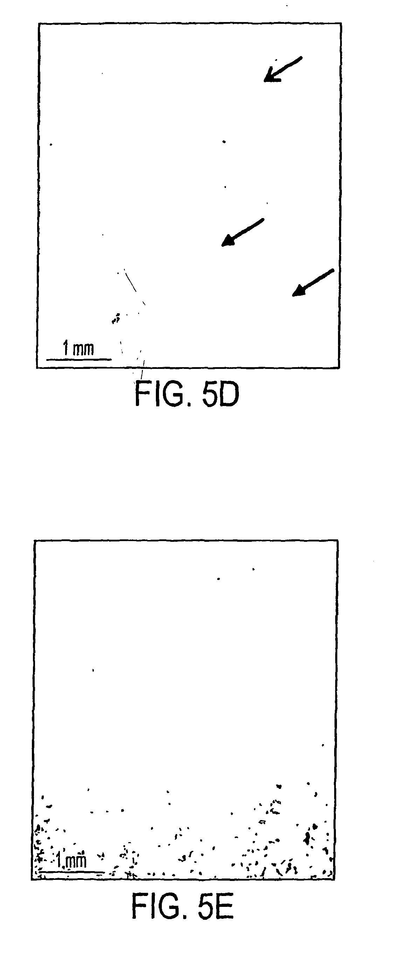

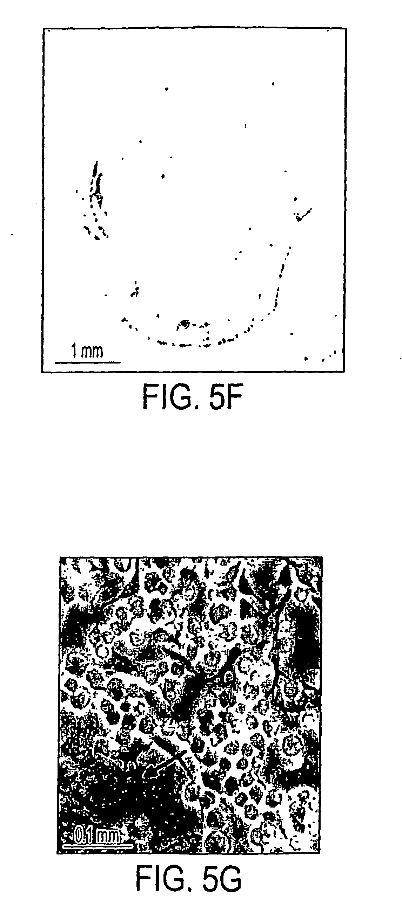

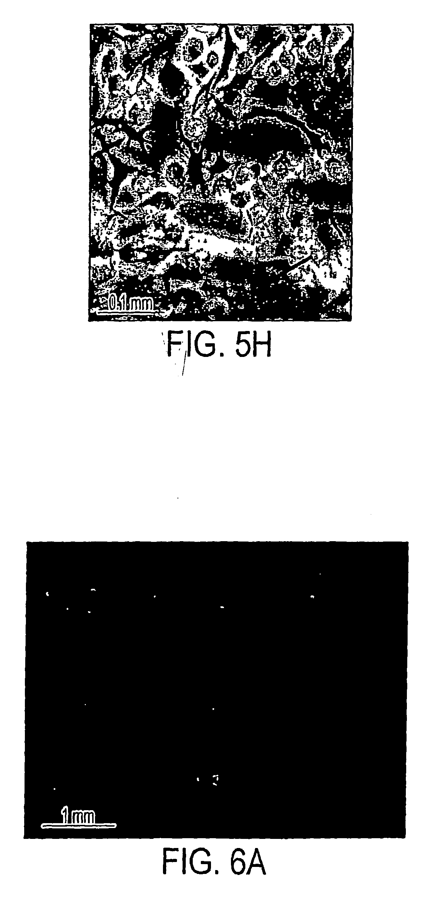

BV-2 microglial cells were cultured in sera free medium on Aα spot (Fig. 5E), Aα spot opsonized by native mAb 196 (Figs. 5D and 5G) and Aα spot opsonized by deglycosylated mAb 196 (Figs. 5F and 5H). For negative control, cells were cultured on blank cover slip (Fig. 5C). Cells were fixed after 24 hours and stained with rat α mouse F4/80 antibody and horseradish peroxidase conjugated Picture Plus polymer. Color reaction was developed with DAB, golden brown color. Aβ spot opsonized with antibody appears as a brown circle (Fig. 5D, hollow arrow) surrounded by small dark brown spots (Fig. 5D, solid arrow) which are the BV-2 cells. Less cells accumulated on Aβ spot opsonized by deglycosylated mAb 196 (Fig. 5D) than on Aβ spot opsonized by native mAb 196 (Fig. 5C). BV-2 cells accumulated on the border of deglycosylated mAb 196 opsonized spot (Fig. 5H) displayed more ramified shape cell morphology, were less stained with anti-F4/80 antibody and exhibited lower cell density at the spot border compared to spot opsonized by native mAb 196 (Fig. 5G). Aβ spot border is indicated by white arrow in Figs. 5G and 5H.

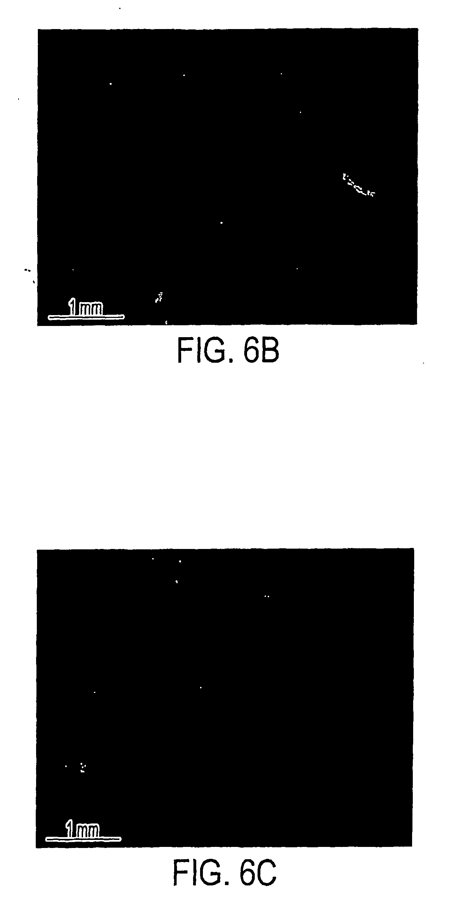

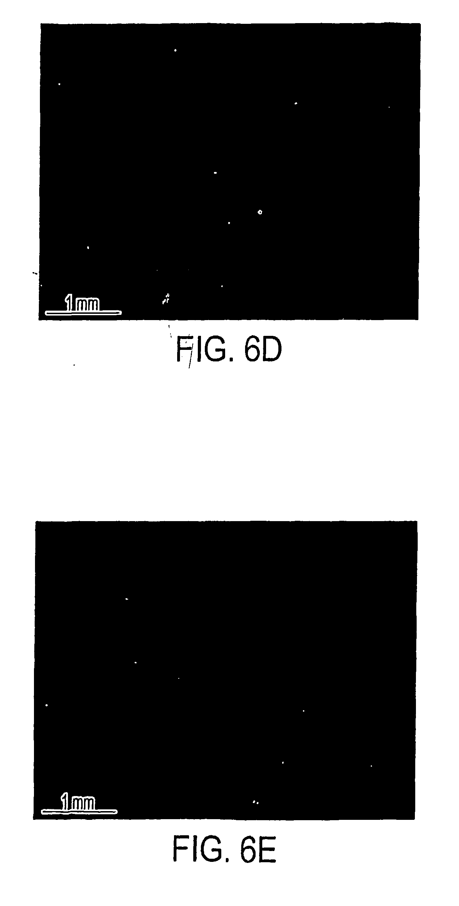

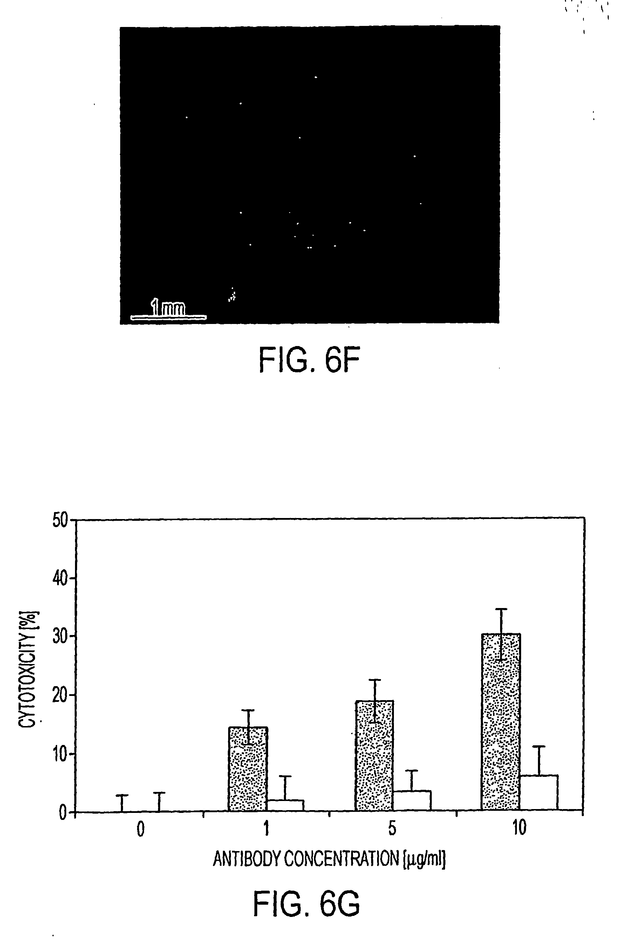

Figures 6A-6G show Fc receptor mediated AβP phagocytosis and ADCC. BV-2 microglial cells were cultured in sera free medium on Aβ spot (Fig. 6B), Aβ spot opsonized by native mAb 196 (Fig. 6C) and Aβ spot opsonized by deglycosylated mAb 196 (Fig. 6D). For negative controls, cells were cultured on blank cover slip (Fig. 6A). In addition, Aβ spot was opsonized with mAb 196 and incubated in absence of BV-2 cells (Fig. 6E) and Aβ spot alone was incubated for the same period of time in absence of BV-2 cells (Fig. 6F). After 24 hours cover slips were stained with Thioflavin S and observed under fluorescent microscope. N2a cells (1000 cells/well) were cultured together with BV-2 cells at 1:2 ratio, respectively (Fig. 6G). Native mAb 196/AβP 1-42 immunocomplex (black) or deglycosylated mAb 196/AβP 1-42 (white) at final antibody concentrations of 1,5 and 10 mg/ml were introduced to BV-2 cells. After 6 hours incubation with the immunocomplex, cell supernatant was collected and LDH release was measured. Spontaneous release was measured in the medium of cells cultured in the assay medium alone, and maximal release was measured in the medium of cells cultured in assay medium supplemented with 2% (v/v) Triton x100. Cytotoxicity value was calculated by reducing spontaneous release (0%) from treated release and dividing it by maximal release value (100%). Error bars represent Standard Deviation in OD 492nm values that were calculated from three independent experiments.

### DETAILED DESCRIPTION OF THE INVENTION

Glycosylation at Asn²⁹⁷ of Fc fragments plays an important role in the binding of Fc fragments to the low affinity receptor FcRIII. The role of carbohydrate appears to be primarily to stabilize the Fc receptor epitope conformation. Besides the normal function of FcR in triggering cellular inflammatory response to clear antigen-bound immune complexes, FcR also mediates autoimmune diseases generated from the response to auto-antibodies, such as the rheumatoid factor in rheumatoid arthritis. Under these conditions, it would be beneficial to block the autoantibody-triggered activation of FcR to relieve the auto-inflammatory response that leads to specific tissue damage. The ability to inhibit receptor activation should, in this case, help to control the antibody-mediated auto-inflammatory response.

All antibodies contain carbohydrate at conserved positions in the constant region of heavy chains, with each isotype possessing a distinct array of N-linked carbohydrate structures. The structures of receptor Fc complexes revealed dominance of lower hinge residues of Fc in the receptor binding, suggesting a new way to inhibit binding of immunoglobulins to their receptors, in order to block Fc receptor activation. The present inventors have now shown that it is possible to produce deglycosylated form of a given monoclonal antibody which preserves the therapeutic function of the native glycosylated monoclonal antibody. Deglycosylated mAb was found to be less efficient in mediating pro-inflammatory response in microglial cell culture due to a reduction in Fc receptor binding. Deglycosylation of monoclonal antibody may allow clearance of Aβ plaques without activating the immune system in brains treated by passive immunization with antibodies, as the clearance of Aβ in vivo was shown to be performed in non-Fc-mediated mechanism (Bacskai et al., 2002). Passive immunization with deglycosylated monoclonal antibody, insofar as CNS disorders are concerned, may present a safer alternative for brain immunotherapy. Modulation of the inhibitory FcR pathway may be an efficient practical therapeutic approach for controlling autoantibody-mediated inflammation induced by self contigens or antibodies in immunotherapeutic strategies for treatment of AD (Figs. 1A and 1B).

The present invention provides an improvement to passive immunization against a disease or disorder characterized by amyloid aggregation by diminishing the risk of triggering or exacerbating an inflammatory response, such as in the case of passive immunization against Alzheimer's disease. The improvement is the use of an unglycosylated antibody against a peptide component of an amyloid deposit, instead of a native glycosylated antibody for preventing, inhibiting or treating a disease or disorder characterized by amyloid aggregation. Such an unglycosylated antibody can be the result of the antibody which is deglycosylated or aglycosylated.

Deglycosylated antibodies can be obtained by enzymatically deglycosylating glycosylated antibodies whereas aglycosylated antibodies can be produced by tunicamycin treatment of antibody producing cells to inhibit the attachment of the oligosaccharide precursor to Asn297 (Jefferis et al., 1989). Preferably, the antibody is an IgG, where the IgG molecule is not glycosylated at residue Asn 297 in the Fc region.

The antibodies and methods for passive immunization against Alzheimer's disease and other diseases or disorders characterized by amyloid aggregation are well known in the art. See for example WO 99/27944 and U.S. Patent 5,688,651, the entire contents of each of which are incorporated herein by reference.

Having now generally described the invention, the same will be more readily understood through reference to the following example which is provided by way of illustration and is not intended to be limiting of the present invention.

### EXAMPLE

Immunotherapy became a strategy for treatment of Alzheimer's disease (AD), by either inducing antibody response to amyloid beta peptide (AβP) or by passive administration of anti-AβP antibodies. Clearance of amyloid plaques involves interaction of immunoglobulin Fc receptor-expressing microglia and antibody-opsonized Aβ deposits, stimulating phagocytosis but may promote neuroinflammation. Carbohydrate moiety of Fc of IgG molecule plays a significant role in modulating binding to Fc receptors and its effector functions. In the studies below, glycan was enzymatically removed from monoclonal antibody 196 raised against AβP. Antigen binding ability and *in vitro* stability of deglycosylated antibody were unaffected by deglycosylation. Moreover, deglycosylated antibody exhibits low affinity to Fc receptors on microglial BV-2 cells, and has limited ability to mediate microglial chemotaxis and antibody-dependent cytotoxicity compared to native antibody. These data suggest deglycosylation of anti-Aβ antibodies prior to in vivo administration might prevent microglial over-activation, thus reducing risk of neuroinflammatory response during passive immunization.

Abbreviations: AD: Alzheimer's disease, AβP: amyloid beta peptide, FcγRI: Fc gamma receptor type 1, C_{H}2: constant heavy chain domain 2, N-linked: asparagine linked, PNGase F: peptide-N-glycosidase-F, mAb196: monoclonal antibody 196,Con A: Concanavalin A lectin, PI: protease inhibitors, MSR: Macrophage scavenger receptors, RO water: reverse osmosis water, ADCC: Antibody Dependent Cellular Cytotoxicity, LDH: lactate dehydrogenase, SPR: Surface Plasmon Resonance, Tg2576: mutated human APP over-expressing transgenic mice, CML: Complement Mediated Lysis.

### Materials and Methods

### Deglycosylation of mAb196

Monoclonal antibody 196 (mAb 196) was deglycosylated by enzymatic digestion with 5U recombinant peptide N-Glycosidase F (PNGase)/1 µg of IgG (PNGase F purified from *Flavobacterium meningosepticum,* New England BioLabs, Beverly, MA) under non-denaturating conditions - 50 mM sodium phosphate, pH 7.5, with protease inhibitor cocktail (Roche, Germany) - for 5 days at 25°C. Deglycosylation rate was followed by terminating the reaction at different time points (2 hours to 5 days). In addition, mAb 196 was deglycosylated under denaturating conditions as a positive control for full deglycosylation reaction. Prior to the PNGase F digestion, mAb 196 was incubated in denaturing buffer (0.5% SDS, 1% β-mercaptoethanol) at 100°C for 10 minutes and then transferred to a reaction buffer (50mM sodium phosphate pH 7.5) supplemented with 1% NP-40. Both denaturing buffer and reaction buffer were supplied with the PNGase F enzyme. Deglycosylated monoclonal antibody 196 was purified on Hi-Trap protein G column (Amersham Biosciences, Sweden) using AKTA *Prime* (Amersham Biosciences, Sweden) continuous chromatography system.

### Gel electrophoresis

PNGase F treated and non-treated antibody was analyzed by electrophoresis using 10% SDS PAGE, as described by Laemmli (1970). Equal amounts of protein of deglycosylated and native IgG (determined by Bradford assay) were loaded on polyacrylamide gel (3 µg per lane). Prestained broad-range molecular weight standards (BioRad, USA) were used as reference. The gel was stained with BioSafe Coomassie (BioRad, USA) according to manufacturer's instructions.

### Concanavalin A blot

Deglycosylated and native mAb 196, and bovine serum albumin (BSA) as negative control, were electrophoresed and electrotransferred to PVDF membrane (PALL Life Sciences, USA) in 25mM Tris, 192mM glycine and 20% methanol at 400 mA for 90 minutes. Excess binding sites were blocked with 1% (w/v) BSA (Amresco, USA) in 50mM Tris, pH 7.5, supplemented with 1mM MgCl₂, 1mM CaCl₂ and 0.1mM MnCl₂ for 1 hour at room temperature. The membrane was washed three times with 50mM Tris pH 7.5 supplemented with 1mM MgCl₂, 1mM CaCl₂ and 0.1mM MnCl₂, and incubated with 0.4 ng/ml horseradish peroxidase conjugated Concanavalin A (Sigma, USA) at 4°C overnight. Blots were developed using Enhanced Chemiluminesence System (ECL) according to the manufacturer's instructions.

### Enzyme Linked Immunosorbent Assay (ELISA).

An amplified enzyme-linked immunosorbent assay (ELISA) (Engvall and Perlman, 1971) was performed in 96-well microtiter plates (NUNC, USA). Following incubation with 1 µg avidin (Sigma, USA) per well and washed, wells were coated with 0.5.µg of biotinylated AβP (1-16) peptide per well. All washes were conducted once with PBST (0.05% Tween 20) and twice with PBS. To reduce non-specific protein adsorption to polystyrene, wells were blocked with 3% (w/v) skim milk (Tnuva, Israel) in PBS for 1 hour at 37°C. After rinsing, 50 µl of serial dilutions of native 196 mAb (3mg/ml) or deglycosylated 196 mAb (3mg/ml) in 1% (w/v) skim milk were added to each well and incubated for 1 hour at 37°C. Unbound mAb was washed out and bound mAb was detected by horseradish peroxidase conjugated goat anti-mouse IgG antibody (Jackson, USA) (1:5000 dilution). The enzyme reaction was visualized by adding 2 gr/ml o-phenylenediamine (Sigma, USA) in 0.05M citric acid pH 5 containing 10% (v/v) hydrogen peroxide. Color development was terminated by adding 1M HCl, 50 µl per well. For quantification, absorbance at 492 nm was measured with 405 nm reference filter using spectrophotometric microplate reader (Sunrise, Tecan, Austria).

### In vitro stability of deglycosylated mAb 196

Deglycosylated mAb 196 (1.5 µg) were spiked into 60 µl normal mouse serum and incubated at 37°C. As controls, deglycosylated mAb 196 was incubated in normal mouse serum with protease inhibitors (PI), PBS and PBS with protease inhibitors. Stability was assayed by AβP 1-16 binding in comparison to native mAb 196 exposed to the same treatments. Activity was measured by ELISA, as described earlier at the starting point of the experiment, after two hours and after 1 week.

### Immunohistochemistry of amyloid beta plaques in brains of APP Tg mice

Brain sections of hAPP Tg mice (Tg2576) were deparaffinized by a series of xylenes and hydrated by decreasing alcohol gradient. Endogenic peroxidase activity was quenched by 3% (v/v) hydrogen peroxide solution in absolute methanol. Antigen retrieval was achieved by microwaving the samples in citrate buffer, 0.01 M, pH 6.0. Non-specific interactions of the antibody with the tissue were blocked by histomouse blocker (Zymed, USA). Blocked brain sections were incubated with mAb 196 and deglycosylated mAb 196 in equal final concentrations of 1µg/ml for one and a half hours at room temperature. Unbound antibody was removed by washes with TBS. Bound mAb was detected by incubation with horseradish peroxidase conjugated Picture Plus polymer (Zymed, USA). Enzyme reaction was generated by incubation with DAB (Zymed, USA). Stained brain sections were viewed using light microscope (Leica DMLB, Germany) and photographed with digital CCD camera. (ProgRes C14, SciTech, Australia).

### Visualization of Fc receptors on microglia cells by:

### (1) Immunofluorescence

Fc receptors (FcR) on microglial BV-2 cells were stained with rat α mouse FcγRII/III (CD16/32) (Southern Biotech, USA) antibody as follows: BV-2 cells were cultured on an eight chamber slide (Nunc, USA) at 4x10⁴ cells per chamber in DMEM (Biological Industries, Israel) supplied with 5% fetal calf serum (FCS) (Biological Industries, Israel) in 90% relative humidity and 5% CO₂ for 5 days. To minimize receptor internalization, staining procedure was carried out in an ice-bath until fixation. Blocking was done by incubation with 3% (w/v) BSA and 10% (v/v) goat normal serum (Jackson, USA) in PBS for 30 minutes. Following washing with ice cold PBS, cells were incubated with rat α mouse FcγRII/III (CD16/32) at 1:20 dilution for one hour at 4°C. Cells were then washed with ice cold PBS and incubated with biotin conjugated goat α rat IgG (Jackson, USA) at 1:20 dilution and FITC conjugated avidin (Jackson, USA) at 1:300 dilution for one hour. Unbound secondary antibody was removed by rinsing the cells with ice cold PBS. Cells were fixed with 4% (w/v) paraformaldehyde for 30 minutes at 4°C and rinsed with PBS. The chambers were disconnected from the glass and mounted with Antifade mounting medium (Molecular Probes, USA). Cell fluorescence was visualized using scanning laser confocal microscopy. Negative control included cells incubated only with the secondary antibody.

### (2) Flow cytometry

Fc receptors on microglial BV-2 cells were visualized by flow cytometer. BV-2 microglial cells were detached from the flask bottom by tapping and collected in FACS vials (5x10⁵ cells per vial). All washes and incubations were conducted with ice cold PBS with 2% BSA and 0.2% (v/v) sodium azide. Cells were washed and incubated for one hour with rat α mouse FcγRII/III (CD16/32) (Southern Biotech, USA) at 1:20 dilution for 45 minutes at 4°C. After washing, the cells were incubated with biotin conjugated goat α rat IgG (Jackson, USA) (1:50) followed by avidin-FITC (1:300) (Jackson, USA) for 45 minutes at 4°C in the dark. Following additional washings, cells were fixed with 4% paraformaldehyde for 30 minutes at 4°C and rinsed with PBS. Negative control included cells incubated only with secondary antibody. Cell fluorescence was measured using FACScan (Becton, USA) with WinMDI software. 5000 Events were accumulated per sample with fluorescence measured on logarithmic scale.

### Binding of immunocomplex 196-Aβ to Fc receptors on microglial cells measured by:

### Immunofluorescence

Immunocomplexes of deglycosylated mAb 196 and preaggregated Aβ 1-42 (Global Peptide Services, USA) were added to BV-2 cultured cells. Bound antibody was detected by Cγ3 conjugated goat α mouse IgG (Jackson, USA) as follows. BV-2 cells were cultured on an eight chamber slide, washed and blocked as described earlier, and incubated for one hour with the immunocomplex of deglycosylated mAb 196 with preaggregated Aβ1-42 peptide for 2 hours at 37°C, at a molar ratio of 1:30. Macrophage scavenger receptors (MSR) were blocked with fucoidan (0.2 mg/ml) (Sigma, USA) to prevent its interaction with Aβ1-42) peptide during the incubation. Unbound immunocomplex was removed by rinsing the cells with ice cold PBS while the immunocomplex was detected with Cγ3 conjugated goat α mouse IgG (Jackson, USA) at 1:500 dilution in 1% (w/v) BSA. Cells were then fixed with 4% (w/v) paraformaldehyde for 30 minutes at 4°C and rinsed with PBS. The chambers were disconnected from the glass and mounted with Antifade mounting medium (Molecular Probes, USA). Cells incubated with native 196-mAb immunocomplex at the same concentration were used as positive control. Negative controls included cells incubated with mAb 196 without the antigen and cells incubated only with the secondary antibody. Fluorescence was observed with light microscope (Leica DMLB, Germany) and photographed with digital CCD camera (ProgRes C14, SciTech, Australia).

Cells that were already stained with mAb 196/ Aβ peptide immunocomplex + Cγ3-goat α mouse IgG (H+L), were double-stained with biotin conjugated rat. α CD16/32 FcR (Southern Biotech, USA) at 1:20 dilution, and FITC conjugated avidin (Jackson, USA) at 1:300 dilution using staining procedure described above. Double-staining results were visualized using scanning laser confocal microscopy.

### FACS analysis

Immunocomplex of mAb 196 and aggregate of Aβ (1-42) was analyzed by FACS. BV-2 cells, cultured in an eight chamber slide, were collected in FACS vials, washed with ice cold PBS with 2% BSA, 0.2% sodium azide and 0.2 mg/ml fucoidan, and incubated for one hour with deglycosylated Aβ (1-42) immunocomplex at a final concentration of 10 µg/ml for 45 minutes at 4°C. Cells were washed to remove unbound material and incubated with Cγ3 conjugated goat α mouse IgG (Jackson, USA) at 1:500 dilution for 45 minutes at 4°C in the dark. After rinsing with ice cold PBS, the cells were fixed with 4% paraformaldehyde for 30 minutes at 4°C followed by washing with PBS. For comparison, cells incubated with native Aβ (1-42) immunocomplex at the same concentration were used. Negative control included cells incubated only with secondary antibody. Cell fluorescence was measured using FACScan (Becton, USA) with WinMDI software. 5000 Events were accumulated per sample with fluorescence measured on logarithmic scale.

### Cell migration assay

Aβ (1-42) (2.5 mg/ml) (Global Peptide Services, USA) was incubated for four days at 37°C. Spots (2 µl) of aggregated Aβ (1-42) were applied onto each 13mm ∅ glass coverslip. After drying, the coverslips were transferred to a 24-well culture plate and incubated for two hours at 37°C with OPTIMEM1 (Gibco, UK) containing 1% FCS to block non-specific binding of antibody to Aβ. After blocking, the Aβ spots were washed with serum free OPTIMEM1 and incubated with 10 µg/ml mAb 196 native and/or deglycosylated forms, or with irrelevant mouse IgG for two hours at 37°C. The coverslips were rinsed with serum free OPTIMEM1 and BV-2 microglial cells were added (50000 cells in OPTIMEM1 per well). At different time intervals, cells were fixed and analyzed by F4/80 immunohistochemistry for migration in the vicinity of the plaques.

### F4/80 immunohistochemistry of microglia activation

BV-2 cells, cultured as described earlier, were fixed with 4% paraformaldehyde for 30 minutes and the activation level evaluated with rat α mouse F4/80 (Serotec, England), a macrophage activation antigen (Gordon, 1995). After fixation, cells were rinsed with PBS and blocked with 3% BSA in PBST for 30 minutes at room temperature. The cells were incubated with rat α-mouse F4/80 (1:100) for two hours at room temperature. To detect rat α-mouse F4/80 and mouse mAb 196 bound to the Aβ spot, horseradish peroxidase conjugated Picture Plus polymer (Zymed, USA) was added for one hour at room temperature. Color reaction was developed equally for all wells with DAB (Zymed, USA).

### Thioflavin S staining

Following F4/80 immunohistochemistry, cells were washed with RO water and stained with 1% (w/v) Thioflavin S (Sigma,USA) for 3 minutes. After staining, cells were thoroughly rinsed with RO water and mounted with GVA mounting medium (Zymed, USA). Thioflavin S induced fluorescence was observed with light microscope (Leica DMLB, Germany) and photographed with digital CCD camera (ProgRes C14, SciTech, Australia).

### Antibody dependent cellular cytotoxicity

To study the effect of Fc deglycosylation on ADCC mediated by microglia, N2-α mouse neuroblastoma cell line and BV-2 mouse microglia cell line were used as target and effector cells, respectively. ADCC was evaluated by measurement of lactate dehydrogenase (LDH) activity in medium (Cytotoxicity detection Kit [LDH], Roche, Germany). N2a cells (1000 cells/well) were cultured in serum free medium.OPTIMEM1 (Gibco, Germany) together with BV-2 cells at 1:2 ratio, respectively. Following overnight incubation, medium was exchanged to fresh medium supplemented with native mAb 196/Aβ 1-42 immunocomplex or with deglycosylated mAb 196/Aβ 1-42 at final antibody concentrations of 1, 5 and 10 µg/ml. After 6 hours incubation with the immunocomplex, supernatant was collected and negative LDH release was measured. Spontaneous LDH release, used as negative control, was measured by adding medium to wells containing N2a and BV-2 cells. Maximum LDH release, used as positive control, was measured after adding medium supplemented with 2% (v/v) Triton X-100 solution to wells containing N2a and BV-2 cells. Supernatant was removed by centrifugation of the microtiter plate at 250g for 10 min, and 100 µl were transferred into corresponding wells of an optically clear 96-well flat-bottom microtiter plate. To determine the LDH activity in these supernatants, 100 µl of reaction mixture (prepared according to manufacturer's instructions) were added to each well and incubated for 15 minutes in the dark at room temperature. The absorbance of the samples was measured at 492 nm with 620nm reference filter using a spectrophotometric microplate reader (Sunrise, Tecan, Austria). ADCC value was calculated as follows: (treated cells release - spontaneous release)/ maximal release x100.

### Results

### Deglycoslylation of anti-Aβ antibody 196

The deglycosylation reaction was conducted under non-denaturating conditions in order to maintain biological activity of the antibody. To overcome poor accessibility of glycans to the enzyme, a long incubation time (up to 5 days), and a large amount of enzyme were used. SDS PAGE analysis (Fig. 2A) showed that after 1.5 hours deglycosylated IgG was generated (lower band at 50kDa) and the intensity of the glycosylated heavy chain (upper band at 50kDa) decreased. As the reaction proceeds, the lower band intensity increases until the upper band becomes undetectable. The end point of the reaction (Fig. 2A, 5 days) showed one band at 50kDa that migrates similarly to the fully deglycosylated heavy chain (Fig. 2A, denatured). Glycosylated mAb 196, deglycosylated mAb196 and BSA as negative control, were blotted onto the PVDF membrane and incubated with a lectin-Concanavalin A (Con A) that binds the core mannose of the glycan (Figs. 2B and 2C). Figure 2C shows ConA binding to the heavy chain of native mAb 196 and almost no binding to the heavy chain of deglycosylated mAb 196. As expected, there was no detectable binding of Con A to BSA, which is a non-glycosylated protein.

### Properties of deglycosylated antibody

*In vitro* stability of deglycosylated mAb196 was tested by measuring its binding to the antigen after long-term incubation in serum (Fig. 3A). No significant difference in OD₄₉₂ₙₘ was observed between native and deglycosylated mAb 196 incubated in serum after one week. A slight time-dependent reduction is noticeable in both, as well as in mAb 196 incubated in serum supplemented with protease inhibitors (PI). Incubation of native or deglycosylated mAb in PBS with PI did not result in decreased antigen binding activity.

Antigen recognition of deglycosylated mAb196 was tested by ELISA against Aβ (1-16) peptide (Fig. 3B) and by Aβ plaque immunohistochemistry (Fig. 3C). ELISA showed that the deglycosylated antibody exhibited only a slight reduction of OD₄₉₂ₙₘ in higher dilutions of the antibody, due to deglycosylation (at 0.3 µg/ml), compared to the glycosylated mAb 196. Immunostaining of Aβ plaques by deglycosylated mAb 196 showed equivalent results both for native and deglycosylated antibody (Fig. 3C).

### Visualization of Fcγ receptors and F4/80 antigen on microglia cells

The BV-2 microglial cell line exhibits similar behavioral characteristics to in vivo microglial cells (Bocchini et al., 1992). FACS analysis showed a significant peak shift on the fluorescence scale in comparison to control, showing FcγIIR/ FcγIIIR expression by BV-2 cells (Fig. 4A). The membrane associated fluorescence was observed when BV-2 cells were stained with anti-FcγIIR/ FcγIIIR antibody (Fig. 4B). These results concur with previous findings that demonstrate expression of Fc receptors on microglial cells (Akiyama et al. 2000; Bard et al. 2000; and Schenk et al. 1999) and confirm the abundance of Fc receptors on BV-2 microglial cells. In addition to Fc receptor expression on BV-2 cells, microglia activation was investigated using F4/80 as an activation marker. F4/80 is a cell surface, antigen which is expressed by mature and activated macrophages (Gordon, 1995). In order to be able to visualize various activation levels, BV-2 cells were cultured in serum free media. Phase contrast image of the cells immunostained with anti F4/80 antibody revealed good correlation between cell morphology and intensity of F4/80 staining (Figs. 5A and 5B). Ramified BV-2 cells appeared as a star-shaped morphology and were weakly stained with anti-F4/80 antibody (Figs. 5A and 5B, hollow arrow). On the other hand, phagocytic microglia displaying round cell morphology, were strongly stained with anti-F4/80 antibody (Figs. 5A and 5B, solid arrow). Visualization of Fc receptors and F4/80 expression in BV-2 murine microglia cell line enabled use of this cell line as a model in this study.

### Microglial activation via Fc receptors by the immunocomplex mAb 196/AβP.

The effect of Aβ immunocomplexation was examined with native and deglycosylated mAb 196 on binding to Fc receptors on BV-2 cells. It has previously been shown, using Surface Plasmon Resonance (SPR) measurements, that deglycosylation of Fc results in reduction of the receptor binding to IgGl (Radaev et al., 2001). The immunocomplex of deglycosylated mAb 196 and Aβ (.1-42) added to BV-2 cells was visualized by immunofluorescence technique. Fluorescent microscopy of BV-2 cells indicated a decline in cell associated fluorescence when deglycosylated immunocomplex was added (Fig. 4F), compared to immunocomplex of the Aβ (1-42) with the native antibody (Fig. 4D). BV-2 cells, incubated with mAb 196 alone as a control (results not shown), showed no fluorescence. FACS analysis showed a 35 percent reduction in mean cell fluorescence when deglycosylated immunocomplex was added to the cells (Fig. 4E) compared with the native one (Fig. 4C). FcγR fluorescence on cells was confirmed by double-labeling with anti-FcγII/IIIR antibody (results not shown). Subsequently, the BV-2 cell activation level was examined when cells were cultured on Aβ spot opsonized with deglycosylated mAb 196. Microglia exhibited pronounced chemotaxis to preaggregated Aβ (1-42) deposits and showed increased migration and chemotaxis to antibody-opsonized spot (Lue et al., 2001 and 2002). Migration and activation of BV-2 cells, due to antibody-opsonization of Aβ (1-42) deposits, were measured on the Aβ spot model. Horseradish peroxidase Picture Plus polymer enabled double-staining with anti-F4/80 antibody and binding of the mAb 196 to the spot simultaneously. Aβ spot opsonized with antibody appears as a brown circle (Fig. 5D, hollow arrow) surrounded by small dark brown spots which are BV-2 cells (Fig. 5D, solid arrow). A series of experiments revealed that after 24 hours, a smaller number of cells migrated to and accumulated on the Aβ spot opsonized by deglycosylated mAb 196 compared to the number of cells which migrated towards the Aβ spot opsonized by native mAb 196. BV-2 cells accumulated on the border of the deglycosylated mAb 196 opsonized spot, displaying a more ramified shaped cell morphology (Fig. 5H). The cells were stained only slightly with anti-F4/80 antibody and exhibited lower cell density at the spot border in comparison to the spot opsonized by native mAb 196 (Fig. 5G). Thus, considering cell morphology and the degree of F4/80 staining, deglycosylation of the spot opsonized antibody reduced BV-2 cell activation. To follow aggregated Aβ spots during the experiment, Thioflavin S staining was performed (Figs. 6A-6F). After 48 hours, there was a noticeable reduction in the green fluorescence of opsonized Aβ spots, whether with native or deglycosylated mAb 196 (Figs. 6C and 6D). This phenomenon was not observed when Aβ spots were incubated with mAb 196 but without BV-2 cells under the same conditions (Fig. 6E), suggesting the phagocytic abilities of BV-2 cells. Over a period of 48 hours, removal of the Aβ spot opsonized with mAb 196 or deglycosylated mAb 196 by BV-2 cells was considerably more efficient than that of the Aβ spot alone (Fig. 6B). Deglycosylation of mAb 196 did not completely abolish Fc mediated phagocytosis of Aβ by BV-2 cells. Additionally, deglycosylated mAb 196 displayed lower ADCC values in comparison to ADCC values displayed by native mAb 196 (up to 30% in a dose response manner to antibody concentrations) (Fig. 6G). At 10 µg/ml antibody concentration, ADCC value measured for deglycosylated mAb 196 was 6-fold lower than ADCC value measured for native mAb 196.

### Discussion

The AD brain is characterized by selective neuronal loss, neurofibrillary tangles, and abundant extracellular deposits of insoluble amyloid protein (Glenner et al. 1984). In particular, the senile plaques of AD are sites of inflammatory processes, as evidenced by the presence of reactive microglia and astrocytes associated with the plaques (Itagaki et al. 1989). It is possible that activation of microglial cells leads to the production of various cytokines and neurotoxins, which may ultimately cause neuronal injury and death (Barger et al., 1997; Egensperger et al. 1998; and Benveniste et al. 2001).

Induction of systemic adaptive immune responses to Aβ peptide in mouse models of AD has been found to be beneficial both neuropathologically and behaviorally (Schenk et al., 1999; and Games et al. 2000). It appears that induction of antibodies to Aβ plays the primary role in the vaccine-mediated clearance of Aβ from the brain, as passive transfer of Aβ antibodies has shown similar beneficial effects (Bard et al. 2000).

However, the antibody-mediated approach by microglial clearance of Aβ may simultaneously drive limited but tangible inflammatory damage to the surrounding tissue.

Modulation of the inhibitory FcR pathway may be an efficient practical therapeutic approach for controlling autoantibody-mediated inflammation induced by self-antigens or antibodies in immunotherapeutic strategies for treatment of AD.

In the study in this example, enzymatic deglycosylation was used to remove the glycan from anti-AβP monoclonal antibody 196. Glycosylation at Asn297 of Fc fragments plays an important role in binding Fc fragments to the low affinity receptor FcγRIII. The role of carbohydrate appears primarily to stabilize the Fc receptor epitope conformation. Removal of carbohydrate results in reduction of FcγRIII binding to IgGl, showing the importance of carbohydrates to FcγRIII function (Radaev et al., 2001). The deglycosylated form of mAb 196 retained *in vitro* stability and antigen recognition functions. Murine microglial cell culture (BV-2) exhibited a 35% reduction in binding of deglycosylated mAb 196 to FcγRII and FcγRIII compared to native mAb. FcRγIII is considered the prototypical pro-inflammatory receptor when FcRII limits the scope and duration of toxic inflammatory factors that have already been produced. The overall reduction of 35% in binding of deglycosylated mAb 196 to FcγRII and FcγRIII that was measured might be of even more impact if FcγRIII alone is considered. Activation of microglia by Aβ is associated with the chemotactic response consistent with extensive clustering of activated microglia at sites of Aβ deposition in the AD brain, suggesting that microglia may phagocytose Aβ fibrils (Terry et al., 1975). Similar processes were reported in cell culture activation of microglia associated with Aβ plaques (Akiyama et al. 2000; and Lue et al. 2001). In cell culture, AD microglia not only migrate to aggregated Aβ deposits but also remove it over a period of 2-4 weeks and the opsonization of Aβ with antibody enhances postmortem AD microglial chemotaxis and activation (Lue et al., 2002). Studies using the spot migration model showed that interaction of microglia with antibody opsonized Aβ plaques could exacerbate the existing inflammation (Lue et al., 2002). The activation level and migration response of murine microglial cells (BV-2) to Aβ spot opsonized with a deglycosylated monoclonal antibody 196 was measured. A decrease in the number of cells that migrated to Aβ spot opsonized with deglycosylated monoclonal antibody 196 was observed. The activation level over a period of 24 hours decreased in comparison to cells that migrated to the Aβ spot opsonized with native monoclonal antibody 196. The overall decrease in microglial activation probably results from poor recognition of deglycosylated antibody by Fc receptors. However, judging by Thioflavin S staining of Aβ spots, deglycosylation of mAb 196 did not abolish phagocytosis of the opsonized Aβ spot. The process of microglial chemotaxis, activation, and phagocytosis of Aβ are all inextricably linked by the same receptors and soluble intermediates. Deglycosylated mAb was found to be less efficient in mediating pro-inflammatory response, as measured by ADCC assay, in mixed neuronal and microglial cell culture due to reduction in Fc receptor binding. Several studies have shown that the clearance of Aβ *in vivo* by immunotherapy is a non-Fc-mediated mechanism (Bacskai et al. 2002; and Das et al. 2003), thus deglycosylation of monoclonal antibody may allow clearance of Aβ plaques without activating the immune system in treated brains. It is suggested here that enzymatic deglycosylation of the antibody can reduce the effector functions, like ADCC, but will not affect its therapeutic function, thus making the passive immunization procedure safer and more suitable for neuronal environment. Passive immunization with deglycosylated monoclonal antibody may allow clearance of Aβ plaques without activating the Fc receptor of microglia in the treated brains. The ability to exploit biorecognition functions of antibodies without triggering activation of effector functions can be applicable in brain immunotherapy in general.

Deglycosylation of therapeutic anti-Aβ monoclonal antibodies prior to administration is expected to prevent microglial over-activation and thus reduce risks of a neuroinflammatory response to passive immunization.

### REFERENCES

Akiyama, H., Arai, T., et al., 2000. Cell mediators of inflammation in the Alzheimer disease brain. Alzheimer Dis Assoc Disord, 14 Suppl 1, S47-53.
Bacskai, B.J., Kajdasz, S.T., et al., 2002. Non-Fc-mediated mechanisms are involved in clearance of amyloid-beta in vivo by immunotherapy. J Neurosci, 22, 7873-7878.
Bard, F., Cannon, C., et al., 2000. Peripherally administered antibodies against amyloid beta-peptide enter the central nervous system and reduce pathology in a mouse model of Alzheimer disease. Nat Med, 6, 916-919.
Barger, S.W. and Harmon, A.D., 1997. Microglial activation by Alzheimer amyloid precursor protein and modulation by apolipoprotein E. Nature, 388, 878-881.
Benveniste, E.N., Nguyen, V.T., et al., 2001. Immunological aspects of microglia: relevance to Alzheimer's disease. Neurochem Int, 39, 381-391.
Bocchini, V., Mazzolla, R., et al., 1992. An immortalized cell line expresses properties of activated microglial cells. J Neurosci Res, 31, 616-621.
Boje, K.M. and Arora, P.K., 1992. Microglial-produced nitric oxide and reactive nitrogen oxides mediate neuronal cell death. Brain Res, 587, 250-256.
Castano, A., Lawson, L.J., et al., 1996. Activation and proliferation of murine microglia are insensitive to glucocorticoids in Wallerian degeneration. Eur J Neurosci, 8, 581-588.
Chao CC, Hu S, Molitor TW, Shaskan EG, and Peterson PK (1992) Activated microglia mediate neuronal cell injury via a nitric oxide mechanism. J Immunol 149:2736-2741.
Chao, C.C., Gekker, G., et al., 1994. Human microglial cell defense against Toxoplasma gondii. The role of cytokines. J Immunol, 152, 1246-1252.
Clynes et al, Cytotoxic antibodies trigger inflammation through Fc receptors, Immunity, 3:21-26 (1995).
Collin, M., Svensson, M.D., et al., 2002. EndoS and SpeB from Streptococcus pyogenes inhibit immunoglobulin-mediated opsonophagocytosis. Infect Immun, 70, 6646-6651.
Dalakas MC, Intravenous immunoglobulin in the treatment of autoimmune neuromuscular diseases: present status and practical therapeutic guidelines, Muscle Nerve 22:1479-1497 (1999).
Das, P., Howard, V., et al., 2003. Amyloid-beta immunization effectively reduces amyloid deposition in FcRgamma-/- knockout mice. J Neurosci, 23, 8532-8538.
Das, P.Golde, T. E.,2003. Open peer commentary regarding Abeta immunization and CNS inflammation by Pasinetti et al. Neurobiol Aging, 23 (5), 671-4; discussion 683-4.
DeMattos, R.B., Bales, K.R., et al., 2001. Peripheral anti-A beta antibody alters CNS and plasma A beta clearance and decreases brain A beta burden in a mouse model of Alzheimer's disease. Proc Natl Acad Sci USA, 98, 8850-8855.
Dodart, J.C., Bales, K.R., et al., 2002. Immunization reverses memory deficits without reducing brain Abeta burden in Alzheimer's disease model. Nat Neurosci, 5, 452-457.
Egensperger, R., Kosel, S., et al., 1998. Microglial activation in Alzheimer disease: Association with APOE genotype. Brain Pathol, 8, 439-447.
Games, D., Bard, F., et al., 2000. Prevention and reduction of AD-type pathology in PDAPP mice immunized with A beta 1-42. Ann N Y Acad Sci, 920, 274-284.
Glenner, G.G., Wong, C.W., et al., 1984. The amyloid deposits in Alzheimer's disease: their nature and pathogenesis. Appl Pathol, 2, 357-369.
Gordon, S., 1995. The macrophage. Bioessays, 17, 977-986.
Itagaki, S., McGeer, P.L., et al., 1989. Relationship of microglia and astrocytes to amyloid deposits of Alzheimer disease. J Neuroimmunol, 24, 173-182.
Jefferis R and mageed RA. The specificity and reactivity of rheumatoid factors with human IgG. Monogr Allergy 26:45-60 (1989)
Jefferis, R., Goodall, M., et al., 1995. Glycosylation of antibody molecules. A small step for structure, a leap for function. Adv Exp Med Biol, 376, 153.
Lemere, C.A., Spooner, E.T., et al., 2003. Evidence for peripheral clearance of cerebral Abeta protein following chronic, active Abeta immunization in PSAPP mice. Neurobiol Dis, 14, 10-18.
Liu et al, "Molecular consequences of activated microglia in the brain: overactivation induces apoptosis", J Neurochem 77:182-189 (2001)
Lue, L.F., Walker, D.G., et al., 2001. Modeling microglial activation in Alzheimer's disease with human postmortem microglial cultures. Neurobiol Aging, 22, 945-956.
Lue, L-F., Walker, DG. Modeling Alzheimer's disease immune therapy mechanisms: interactions of human postmortem microglia with antibody-opsonized amyloid beta peptide. J. Neurosc. Res. 70:599-610 (2002).
Lund, J., Takahashi, N, Pound, JD, Goodall, M., Jefferis, R. Multiple interactions of IgG with its core oligosaccharide can modulate recognition by complement and human FcΥ receptor 1 and influence the synthesis of its oligosaccharide chains. J. Immunol. 4964-4969 (1996).
McGuire SO, Ling ZD, Lipton JW, Sortwell CE, Collier TJ, and Carvey PM (2001) Tummor necrosis factor alpha is toxic to embryonic mesencephalic dopamine neurons. Exp Neurol 169:219-230.
Nicoll JA, Wilkinson D, Holmes C, Steart P, Markham H, Weller RO,2003. Neuropathology of human Alzheimer disease after immunization with amyloid-beta peptide: a case report. Nat Med, 9(4), 448-452.
Nose, M. and Wigzell, H., 1983. Biological significance of carbohydrate chains on monoclonal antibodies. Proc Natl Acad Sci USA, 80, 6632-6636.
Radaev S., Sun, PD. Recognition of IgG by FcΥ Receptor. The role of Fc glycosylation and the binding of peptide inhibitors. J. Biolog. Chem. 276(19): 16478-16483 (2001).
Schenk et al, Immunization with amyloid-beta attenuates Alzheimer-disease-like pathology in the PDAPP mouse, Nature, 400 (6740) : 116-117 (1999).
Solomon, B., Koppel, R., et al., 1997. Disaggregation of Alzheimer beta-amyloid by site-directed mAb. Proc Natl Acad Sci USA, 94, 4109-4112.
Stangel et al, Mechanisms of high-dose intravenous immunoglobulins in demyelinating diseases, Arch Neurol 56:661-663 (1999).
Sylvestre et al, Fc receptors initiate the Arthus reaction: redefining the inflammatory cascade, Science, 265:1095 (1994).
Terry RD, W.H., 1975. Neurological and sensory disorders in the elderly. Stratton, New York.
van der Meché and van Doorn, The current place of high-dose immunoglobulins in the treatment of neuromuscular disorders, Muscle Nerve 20:136-147 (1997).
Wilcock, D.M., DiCarlo, G., et al., 2003. Intracranially administered anti-Abeta antibodies reduce beta-amyloid deposition by mechanisms both independent of and associated with microglial activation. J Neurosci, 23, 3745-3751.
Wilcock, D.M., Gordon, M.N., et al., 2001. Number of Abeta inoculations in APP+PS1 transgenic mice influences antibody titers, microglial activation, and congophilic plaque levels. DNA Cell Biol, 20, 731-736.
Wilcock, D.M., Rojiani, A., et al., 2004. Passive amyloid immunotherapy clears amyloid and transiently activates microglia in a transgenic mouse model of amyloid deposition. J Neurosci, 24, 6144-6151.

## Claims

1. Use of an unglycolysated antibody against beta amyloid peptide for manufacturing a medicine for prevention, inhibition or treatment of a disease or disorder, which is **characterized by** amyloid aggregation, wherein said antibody is unglycosylated in the Fc region, and diminishes the risk of neuroinflammation.

2. The use according to claim 1, wherein said unglycosylated antibody is deglycosylated or aglycosylated.

3. The use according to claim 1, wherein said antibody is an IgG molecule.

4. The use according to claim 3, wherein said IgG molecule is not glycosylated at residue Asn 297 in the Fc region.

5. The use according to claim 1, wherein the disease or disorder, which is **characterized by** amyloid aggregation, is Alzheimer's disease.

6. An unglycosylated antibody against beta amyloid peptide for use in preventing, inhibiting or treating a disease or disorder, which is **characterized by** amyloid aggregation, wherein said antibody is unglycosylated in the Fc region, and diminishes the risk of neuroinflammation.

7. An unglycosylated antibody according to claim 6, wherein said unglycosylated antibody is deglycosylated or aglycosylated.

8. An unglycosylated antibody according to claim 6, wherein said antibody is an IgG molecule.

9. An unglycosylated antibody according to claim 8, wherein said IgG molecule is not glycosylated at residue Asn 297 in the Fc region.

10. An unglycosylated antibody according to claim 6, wherein the disease or disorder, which is **characterized by** amyloid aggregation, is Alzheimer's disease.

## Patentansprüche

1. Verwendung eines unglykosylierten Antikörpers gegen Beta-Amyloidpeptid in der Herstellung einer Medizin zur Prävention, Hemmung oder Behandlung einer Krankheit oder Erkrankung, die durch eine Amyloidaggregation **gekennzeichnet** ist, wobei der Antikörper in der Fc Region unglykosyliert ist und das Risiko einer Nervenentzündung vermindert.

2. Verwendung nach Anspruch 1, wobei der unglykosylierte Antikörper deglykosyliert oder aglykosyliert ist.

3. Verwendung nach Anspruch 1, wobei der Antikörper ein IgG-Molekül ist.

4. Verwendung nach Anspruch 3, wobei das IgG-Molekül bei Rest Asn 297 in der Fc Region nicht glykosyliert ist.

5. Verwendung nach Anspruch 1, wobei die Krankheit oder Erkrankung, die durch eine Amyloidaggregation **gekennzeichnet** ist, Alzheimer-Krankheit ist.

6. Unglykosylierter Antikörper gegen Beta-Amyloidpeptid zur Verwendung in der Prävention, Hemmung oder Behandlung einer Krankheit oder Erkrankung, die durch eine Amyloidaggregation **gekennzeichnet** ist, wobei der Antikörper in der Fc Region unglykosyliert ist und das Risiko einer Nervenentzündung vermindert.

7. Unglykosylierter Antikörper nach Anspruch 6, wobei der unglykosylierte Antikörper deglykosyliert oder aglykosyliert ist.

8. Unglykosylierter Antikörper nach Anspruch 6, wobei der Antikörper ein IgG-Molekül ist.

9. Unglykosylierter Antikörper nach Anspruch 8, wobei das IgG-Molekül bei Rest Asn 297 in der Fc Region nicht glykosyliert ist.

10. Unglykosylierter Antikörper nach Anspruch 6, wobei die Krankheit oder Erkrankung, die durch eine Amyloidaggregation **gekennzeichnet** ist, Alzheimer-Krankheit ist.

## Revendications

1. Utilisation d'un anticorps non glycosylé dirigé contre le peptide bêta amyloïde pour la fabrication d'un médicament destiné à la prévention, à l'inhibition ou au traitement d'une maladie ou d'un trouble, qui est **caractérisé**(e) par l'agrégation amyloïde, ledit anticorps étant non glycosylé dans la région Fc, et diminuant le risque de neuro-inflammation.

2. Utilisation selon la revendication 1, ledit anticorps non glycosylé étant déglycosylé ou aglycosylé.

3. Utilisation selon la revendication 1, ledit anticorps étant une molécule d'IgG.

4. Utilisation selon la revendication 3, ladite molécule d'IgG étant non glycosylée au niveau du résidu Asn 297 dans la région Fc.

5. Utilisation selon la revendication 1, la maladie ou le trouble, qui est **caractérisé**(e) par l'agrégation amyloïde, étant la maladie d'Alzheimer.

6. Anticorps non glycosylé dirigé contre le peptide bêta amyloïde destiné à être utilisé dans la prévention, l'inhibition ou le traitement d'une maladie ou d'un trouble, qui est **caractérisé**(e) par l'agrégation amyloïde, ledit anticorps étant non glycosylé dans la région Fc, et diminuant le risque de neuro-inflammation.

7. Anticorps non glycosylé selon la revendication 6, ledit anticorps non glycosylé étant déglycosylé ou aglycosylé.

8. Anticorps non glycosylé selon la revendication 6, ledit anticorps étant une molécule d'IgG.

9. Anticorps non glycosylé selon la revendication 8, ladite molécule d'IgG étant non glycosylée au niveau du résidu Asn 297 dans la région Fc.

10. Anticorps non glycosylé selon la revendication 6, la maladie ou le trouble, qui est **caractérisé**(e) par l'agrégation amyloïde, étant la maladie d'Alzheimer.
